# EUROPÄISCHE PATENTSCHRIFT

(11) **EP 1 839 563 B1**
(45) Veröffentlichungstag und Bekanntmachung des Hinweises auf die Patenterteilung: **06.03.2013**
(21) Anmeldenummer: 07006433.2
(22) Anmeldetag: 28.03.2007
(51) Int. Cl.: A61B 1/055, G02B 23/24

(54) **Stablinse zum Einbau in Endoskope**
Rod lens for fitting into endoscopes
Lentille cylindrique destinée à l'installation dans un endoscope

(30) Priorität: 31.03.2006 EP 06006841
(43) Veröffentlichungstag der Anmeldung: 03.10.2007
(73) Patentinhaber: Karl Storz GmbH & Co. KG, 78532 Tuttlingen (DE)
(72) Erfinder: Lederer, Frank, 78532 Tuttlingen (DE); Fürst, Frank, 78532 Tuttlingen (DE); Huber, Matthias, 78576 Emmingen-Liptingen (DE); Rudischhauser, Jürgen, 78532 Tuttlingen (DE)
(74) Vertreter: Weller, Wolfgang

(56) Entgegenhaltungen:
- EP-A- 0 587 177
- EP-A- 1 152 276
- WO-A-95/35522
- US-A- 5 568 312
- US-A- 5 701 200
- US-B1- 6 212 015
- PATENT ABSTRACTS OF JAPAN Bd. 012, Nr. 166 (P-704), 19. Mai 1988 (1988-05-19) & JP 62 279309 A (OLYMPUS OPTICAL CO LTD), 4. Dezember 1987 (1987-12-04)
- NUSIL SILICONE TECHNOLOGY: "MED.6010 Product Profile" [Online] 10. September 2004 (2004-09-10), NUSIL SILICONE TECHNOLOGY , INTERNET , XP002393725 Gefunden im Internet: URL:http://www.nusil.com/PDF/PP/MED-6010P. pdf> [gefunden am 2006-08-07] * das ganze Dokument *
- SUMMERS OPTICAL: 'UV Curing Optical Cements', [Online] 06 Februar 2002, Gefunden im Internet: <URL:http://www.optical-cement.com/cements/ cements/uv.html#anchor42289> [gefunden am 2011-11-14]

## Beschreibung

Die vorliegende Erfindung betrifft eine Stablinse zum Einbau in Endoskope, mit einem stabförmigen Körper, der zumindest zwei Elemente aus Glas aufweist, die jeweils durch ein flexibles, transparentes, massives Zwischenstück zu dem stabförmigen Körper zusammengesetzt sind.

Eine derartige Stablinse ist aus der JP 62 279 309 A bekannt.

Dort sind Stablinsen beschrieben, die zur Gewichtsersparnis einen zylindrischen transparenten Kunststoffkörper beispielsweise aus Polymethylmethacrylat aufweisen. Mittels eines Klebstoffes können an den Kunststoffkörpern Linsen aus Glas angefügt werden. Sofern die Flexibilität der Stablinse angesprochen ist, wird auf eine Knochenform verwiesen. Der zylindrische Kunststoffkörper ist einfacher durch spanabhebende Bearbeitung zu einer zu Stablinse zu formen als Glasmaterialien.

Aus der US 5 701 200 A ist eine Stablinse bekannt, die aus einer Vielzahl an konvexen und konkaven Linsen aus unterschiedlichen Polymermaterialien zusammengesetzt ist. Bei der Herstellung werden die konkaven Linsen im Abstand zueinander in einer Halterung aufgereiht, und die Zwischenräume werden durch ein thermoplastisches, transparentes Kunststoffmaterial aufgefüllt, wodurch sich dann zwischen zwei benachbarten konkaven Linsen entsprechend konvex ausgebildete Linsenkörper bilden.

Als Materialien werden Polycarbonate, Polyacrylate, Polystyrole und ein Material mit der Bezeichnung "VTC-2" eingesetzt. Das Kunststoffmaterial VTC-2 ist ein härtbarer, ausgänglich zähflüssiger Kunststoff, der ausgezeichnete Adhäsionseigenschaften gegenüber Glas, Keramik und verschiedenen Metallen aufweist. Nach dem Aushärten ist dieses Material starr.

Aus dem Datenblatt NUSIL SILICONE TECHNOLOGY "MED-6010" ist ein "Optically Clear Potting and Encapsulating Silicone Elastomer" beschrieben.

Aus US 5 568 312 A ist ebenfalls eine Stablinse bekannt, bestehend aus drei Glaselementen verschiedener brüchiger Materialien (Polycarbonate und BK7 Glas), die mittels eines massiven Stücks optische Zements (VTC-2) verbunden sind. Die Verbindungsstücke sind hierbei jedoch als plan-konvexe Linsen ausgeformt und dienen in Kombination mit den Glaselementen der Reduktion chromatischer und geometrischer Abbildungsfehler. Auf die Elastizität der Stablinse wird kein Bezug genommen.

Endoskope werden allgemein in der minimal-invasiven Chirurgie dazu verwendet, Körperhohlräume und Hohlorgane zu inspizieren.

Endoskope weisen einen langerstreckten Endoskopschaft auf, in dem Bauelemente eines optischen Systems angeordnet sind. Ferner sind im Inneren des Schafts weitere Vorrichtungen, meist eine Lichtzuführungsvorrichtung und Kanäle für Instrumente, Spülflüssigkeit oder dergleichen, vorgesehen. Die Lichtzuführung erfolgt meist über lichtleitende Glasfasern.

Im Jahre 1953 entwickelte und produzierte die Anmelderin ihr erstes Endoskop mit einem traditionellen Linsensystem.

Mit der Erfindung des sog. Hopkins-Stablinsen-Systems ( Katalog der Karl Storz GmbH & Co. KG, Tuttlingen "STORZ, DIE WELT DER ENDOSKOPIE, TECHNISCHE ENDOSKOPIE, 9. Ausgabe 1/2005), das Karl Storz in Zusammenarbeit mit Prof. H. H. Hopkins entwickelte, gelang der entscheidende Durchbruch seit der Entwicklung des konventionellen Linsensystems durch Nitze im Jahre 1879.

Bei Stablinsensystemen nach Hopkins werden anstelle der konventionellen Linsen lange Stablinsen aus Glas verwendet. Diese Stablinsen haben den Vorteil, dass damit Licht mit einer deutlich höheren Effizienz z.B. in einem Endoskop geleitet werden kann, als dies mit herkömmlichen Linsen möglich ist, zwischen denen ein relativ großer Luftraum vorhanden ist.

Dadurch erfüllt das Hopkins-Stablinsen-System die höchsten Anforderungen der medizinischen Diagnostik auf Grund seiner im Vergleich zu dem traditionellen Linsensystem deutlich höheren Lichtübertragung, exzellenten Bildhelligkeit, und wirklichkeitsgetreuen Darstellung.

Zugleich wurde es möglich, den Durchmesser der Endoskope entscheidend zu verkleinern. Insbesondere durch diese Entwicklung fanden die Endoskope mit dem Hopkins-Stablinsen-System eine breite Anwendung in der schonenden, minimal-invasiven Chirurgie.

Wie eingangs beschrieben ist das optische System starrer Endoskope meist in einem innerhalb des Endoskopschafts liegenden Rohr aufgenommen. Endoskopschäfte sind gegenüber ihrem Durchmesser sehr lang, übliche Durchmesser liegen im Bereich von einigen Millimetern, die Länge im Bereich von mehreren Dezimetern. Entsprechend ist der Durchmesser einer Stablinse sehr klein gegenüber deren Länge, die mehrere Zentimeter beträgt.

Es lässt sich bei der Handhabung der medizinischen Endoskope nicht vermeiden, dass deren Schäfte auf Biegung beansprucht werden. Die Biegung der dünnen und langen Endoskopschäfte kann dazu führen, dass es zu Absplitterungen oder sogar zum Bruch der relativ langen Stablinsen aus Glasmaterial kommen kann. Solche Schäden machen ein optisches System unbrauchbar und im Allgemeinen muss das gesamte optische System ausgetauscht werden.

Aus der DE 31 13 110 C2 ist beschrieben, den Stablinsen eine Knochenform zu verleihen, um nur endseitige Anlagepunkte zu schaffen und dadurch die Bruchgefahr zu reduzieren.

Ein weiterer Versuch ist in der US 4,148,551 beschrieben.

Mehrere Stablinsen, die in dem im Endoskopschaft liegenden Rohr angeordnet sind, sind durch einen flexiblen Schlauch miteinander verbunden. Der flexible Schlauch verbindet zwei benachbarte Stablinsen miteinander.

Die Möglichkeit, den Endoskopschaft in gewissen Grenzen zu verbiegen, ohne dadurch die Stablinsen zu brechen, wird durch die flexible Verbindung der Stablinsen mittels des Schlauchs allerdings nur teilweise erreicht.

Wirken Biegemomente direkt auf die einzelne Stablinse ein, kann die Stablinse dennoch brechen. Solche punktuell einwirkenden Biegemomente können auftreten, wenn ein Endoskopschaft beim Handhaben auf eine Kante stößt oder herabfällt.

Aus der WO 95/35522 ist es bekannt, unter anderem flüssige Stablinsen herzustellen, die aus einer stabilen Umhüllung und einem weichen Kern bestehen.

Vor diesem Hintergrund ist es Aufgabe der vorliegenden Erfindung, eine Stablinse der eingangs genannten Art zu schaffen, die die Biegemomente, insbesondere punktuell einwirkende Biegemomente, aufnehmen kann, die während des Gebrauchs eines Endoskops auftreten, ohne dass die Stablinse selbst beeinträchtigt wird, insbesondere nicht bricht.

Erfindungsgemäß wird die Aufgabe durch die Merkmalskombination des Anspruchs 1 gelöst.

Diese Maßnahme hat den Vorteil, dass durch dieses flexible transparente massive Stück der Stablinse insgesamt eine Flexibilität verliehen wird, wodurch die Bruchgefahr des gesamten Stablinsenkörpers stark herabgesetzt wird. Das Kunststoffmaterial ist transparent und hat optische Eigenschaften, die sehr nahe an Glas liegen. Überraschenderweise wurde nun festgestellt, dass dieses Material dazu herangezogen werden kann, um Stablinsen mit hoher Durchlässigkeit und guter optischer Qualität herzustellen. Stablinsen sind ja Körper, deren Länge ein Vielfaches gegenüber deren Durchmesser beträgt. Aus diesem Kunststoffmaterial kann ein massives Stück hergestellt werden, das wie eine Stablinse gehandhabt werden kann, das aber eine gegenüber Glas erhöhte Flexibilität aufweist.

Die Flexibilität bzw. die Elastizität des Kunststoffmaterials ist dabei deutlich höher als die von Glas. Die Härte des Kunststoffmaterials liegt im Bereich von Materialien mit Werten der Shore Härte D kleiner 80 und weicher. Die Reißdehnung sollte mindestens bei 5 % liegen. Die Werte sind abhängig von der Dicke des Materials und der benötigten Durchbiegung der Stablinse. Je kleiner die Dicke bzw. Schichtdicke, desto weicher muss das Material gewählt werden und desto größer muss die Reißdehnung des Materials sein. Silikonmaterialien sind dafür besonders geeignet.

So wird die Gefahr eines Zerbrechens oder Zersplitterns der Stablinse unter den üblichen Belastungen eines starren Endoskops, die sowohl während der Handhabung, z.B. während der Operation oder beim Ablegen des Endoskops, als auch während der Reinigung, bspw. durch Kontakt mit weiteren zu reinigenden Geräten oder während der Reinigung im Ultraschall, auf den Endoskopschaft ausgeübt werden, deutlich verringert, da der Stablinsenkörper eine Flexibilität bzw. Biegbarkeit aufweist.

Das flexible Zwischenstück sorgt für einen Schutz der Stablinse gegenüber äußeren seitlichen Krafteinwirkungen, da die Verbindungen sehr elastisch sind und Stöße daher dämpfen können. So wird die Gefahr eines Zerbrechens der Glaskörper der Stablinsen auch bei einem heftigen Ablegen oder sogar Herabfallen des Endoskops erheblich verringert, da ein Abbiegen oder Abknicken der starren Glaskörper um das flexible Verbindungsstück in einem gewissen Maß möglich ist.

Dadurch, dass das erfindungsgemäß vorgesehene, flexible Zwischenstück auch transparent ist, werden die Lichtübertragung, die Bildhelligkeit und der Kontrast nicht beeinträchtigt.

In einer Ausgestaltung der Erfindung ist der Körper aus drei Elementen zusammengesetzt, wobei zwei benachbarte Elemente jeweils durch ein Zwischenstück miteinander verbunden sind.

Diese Maßnahme hat den Vorteil, dass die Flexibilität einer Stablinse, die aus mehreren, kürzeren, stabförmigen Elementen aus Glas aufgebaut ist, wobei zwei benachbarte Elemente jeweils durch ein Zwischenstück miteinander verbunden sind, sich erheblich erhöht.

Eine derartig konstruierte Stablinse weist eine deutlich höhere Stabilität gegen punktuell einwirkende Biegemomente und Verbiegungen des Endoskopschafts im Vergleich mit der Stabilität der konventionellen Stablinse, die die gleiche Länge aufweist, auf.

Dadurch wird die Gefahr eines Zerbrechens der Stablinse sogar bei einem sehr extremen Biegen eines Endoskops erheblich verringert.

Bei der Erfindung ist das flexible Stück als ein hochflexibler optischer Kitt ausgebildet.

Diese Maßnahme hat den Vorteil, dass der Kitt die Elemente nicht nur aneinander haftend verbindet, sondern nicht streuend ist und die Lichtübertragung, die Bildhelligkeit und den Kontrast nicht beeinträchtigt. Somit können zusätzlich Klebverbindungen über gebräuchliche Klebstoffe entfallen, da der flexible Kunststoff selbst als Klebemittel dient.

Bei der Erfindung sind die Verbindungsstellen der Elemente planar.

Diese Maßnahmen haben den Vorteil, dass bei planaren Grenzflächen zwischen Kitt und Glas die Strahlführung des Lichts unverändert bleibt und eine Neuberechnung der vorhandenen Linsen sich erübrigt.

In einer weiteren Ausgestaltung der Erfindung weist der Körper eine Knochenform auf.

Diese Maßnahme hat den Vorteil, dass die Knochenform als solche an sich schon weniger bruchanfällig ist.

Wird ein solcher knochenförmiger Körper erfindungsgemäß zumindest abschnittsweise durch das flexible Kunststoffmaterial ersetzt oder gar vollständig daraus hergestellt, erhöht sich die Flexibilität noch beträchtlich, so dass selbst bei massivsten Erschütterungen eine Beschädigung der Stablinse auszuschließen ist.

In einer Ausgestaltung der Erfindung sind bei einem Endoskop in einem innerhalb des Schafts liegenden Optikrohrs eine oder mehrere erfindungsgemäße Stablinsen aufgenommen.

Es versteht sich, dass die vorstehend genannten und die nachstehend noch zu erläuternden Merkmale nicht nur in den angegebenen Kombinationen, sondern auch

in anderen Kombinationen einsetzbar sind.

Die Erfindung wird nachfolgend anhand eines ausgewählten Ausführungsbeispiels in Zusammenhang mit den Zeichnungen näher beschrieben und erläutert. Es zeigen:
- Fig. 1: einen Längsschnitt einer Stablinse, deren Körper eine Knochenform hat und zwei Elemente aus Glas aufweist,
- Fig. 2: einen Längsschnitt einer Stablinse, die drei Elemente aus Glas aufweist,
- Fig. 3: eine teilweise geschnittene Seitenansicht eines Endoskops mit erfindungsgemäßen Stablinsen, und
- Fig. 4: einen stark vergrößerten Abschnitt des Endoskopes von Fig. 3 mit gebogenem Schaft.

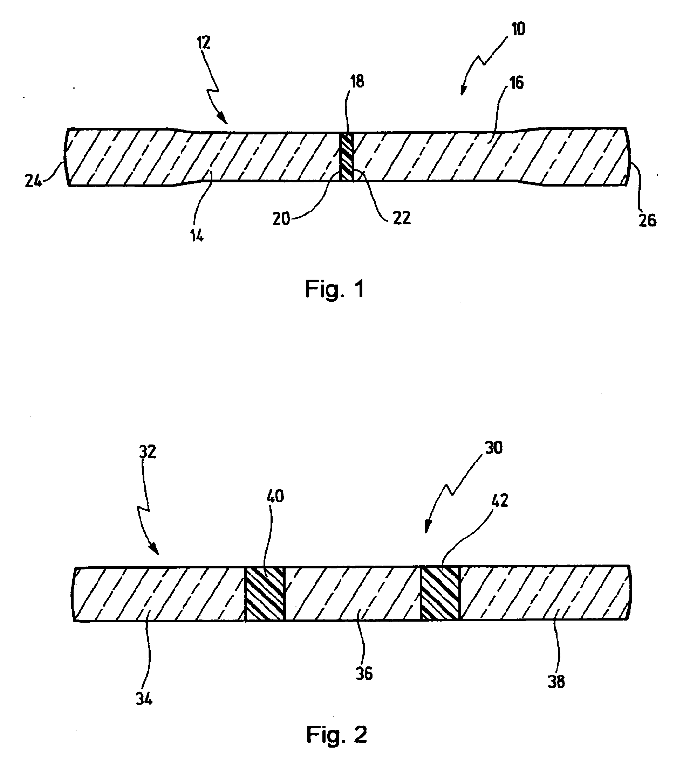

Die in Fig. 1 dargestellte Stablinse in ihrer Gesamtheit mit der Bezugsziffer 10 bezeichnet.

Die Stablinse 10 weist einen knochenförmigen stabförmigen Körper 12 auf.

Der Körper 12 ist aus zwei, etwa gleich großen, spiegelsymmetrischen Elementen 14 und 16 zusammengesetzt, und zwar über ein flexibles, transparentes, massives Zwischenstück 18.

Die beiden Elemente 14 und 16 sind aus Glas hergestellt und können so entstehen, dass man eine herkömmliche knochenförmige Stablinse 10 in der Mitte durch einen Querschnitt auftrennt. Dadurch entstehen zwei planare, sich gegenüber stehende Verbindungsstellen 20 und 22. An ihren äußeren Endseiten sind die Elemente 14 bzw. 16, wie das üblich ist, mit optisch aktiven Flächen versehen, hier mit konvexen Flächen 24 bzw. 26.

Das Zwischenstück 18 besteht aus einem aushärtbaren Kitt, der auch im ausgehärteten Zustand flexibel ist. Dadurch wird die Möglichkeit geschaffen, dass die in Fig. 1 über das Zwischenstück 18 verbundenen Elemente 14 und 16 in einem gewissen Maß bewegt werden können. Dies kann z.B. in einem Verschwenken um das Zwischenstück 18 bestehen. Der ausgehärtete Kitt weist eine Wärmebeständigkeit auf, die den Temperaturen, die bei der Sterilisation eines Endoskops im Autoklaven erfolgen, standhält. Ferner weist der Kitt eine ausreichende Festigkeit, Dehnbarkeit und Zugfestigkeit auf, so dass die Verbindung der gegenüberstehenden Verbindungsstellen 20 und 22 auf Dauer fest ist. Eine weitere wichtige Eigenschaft des Kitts ist die Transparenz. Der Kitt ist nicht streuend und ist blasenfrei zu verarbeiten. Dadurch erfolgt keine Beeinträchtigung der Lichtübertragung, der Bildhelligkeit und des Kontrastes durch das Zwischenstück 18.

Derartige Kitte, die die zuvor genannten Eigenschaften aufweisen, sind von der Firma DELO Industrieklebstoffe GmbH & Co. KG, Landsberg, Deutschland, unter der Bezeichnung "Katiobond 3019" erhältlich.

Bei dem in Fig. 2 dargestellten weiteren Ausführungsbeispiel weist eine Stablinse 30 einen zylindrischen Körper 32 auf. Der zylindrische Körper 32 ist aus drei Elementen 34, 36 und 38 aus Glas zusammengesetzt, wobei dazu zwei Zwischenstücke 40 und 42 dienen. Das mittlere Element 36 ist als zylindrischer Körper mit planaren Endflächen oder Verbindungsstellen ausgebildet. Dieses mittige Element 36 ist über die beiden Verbindungsstücke 40 und 42 mit den jeweils äußeren Verbindungselementen 34 und 38 verbunden, und zwar über planare Verbindungsstellen.

Die jeweils äußere Endfläche der Elemente 34 und 38 ist konvex ausgebildet.

Die Zwischenstücke 40 und 42 sind ebenfalls aus dem zuvor beschriebenen Kitt hergestellt. Durch das Vorsehen von zwei flexiblen Stellen resultiert eine noch höhere Flexibilität bzw. Widerstandskraft gegenüber Biege- oder Scherbeanspruchungen. Mehrere Verbindungsstellen werden dann eingesetzt werden, wenn es sich um extrem lange Stablinsen handelt.

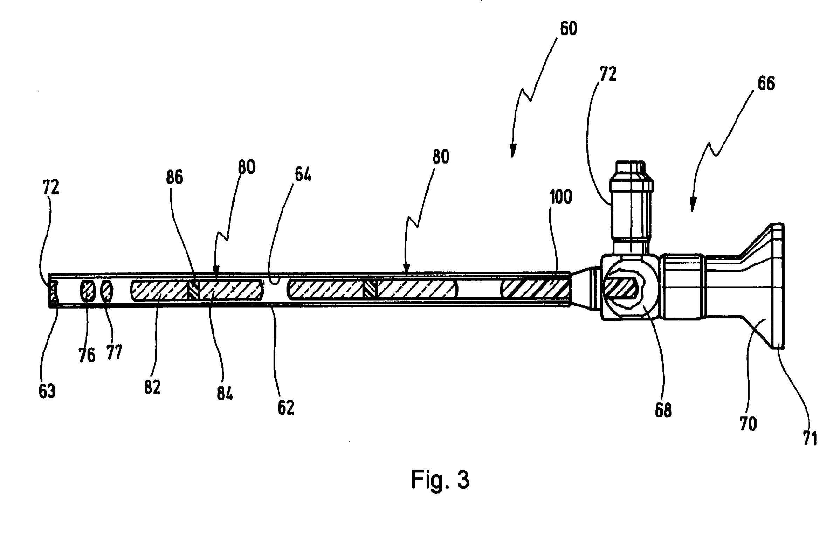

In Fig. 3 ist ein Endoskop 60 dargestellt.

Das Endoskop 60 weist einen Schaft 62 auf, in dem ein inneres Optikrohr 64 aufgenommen ist, das zur Aufnahme von einem optischen System dient. Der Schaft 62 ist mit einem Kopf 66 verbunden, der ein Gehäuse 68, ein endseitiges Okular 70 sowie einen seitlich abstehenden Lichtleiteranschluss 72 aufweist, wie das im Endoskopbau üblich ist.

Endseitig ist der Schaft 62 über ein Fenster 72 abgeschlossen, Linsen 76 und 77 bilden einen distalen Endabschnitt des optischen Systems.

In dem Optikrohr 64 sind zwei Stablinsen 80 aufgenommen. Die Stablinsen 80 sind aus zwei Elementen 82 und 84 aus Glas aufgebaut, die über ein Zwischenstück 86 zusammengesetzt sind. Eine hochflexible Linse 100 liegt im kritischen Übergangsbereich von Endoskopschaft 62 zum Gehäuse 66.

Aus der Seitenansicht von Fig. 3 ist deutlich zu erkennen, dass vom Kopf 66 des Endoskops 60 Bauteile vorstehen, die weit über den Außendurchmesser des Schaftes 62 hinausreichen.

Fällt bspw. das in Fig. 3 dargestellte Endoskop 60 in dieser Ausrichtung auf einen Grund, so trifft zunächst der äußere Rand 71 des Okulares 70 auf den Grund. Dadurch verkippt das Endoskop 60 etwas und der Schaft 62 wird dann mit seinem distalen Ende 63 auf den Grund aufschlagen.

Dabei wirken erhebliche Biegemomente auf den Schaft 62 ein, die den Schaft 62 krümmen. Insbesondere neigen diese Kräfte dazu, den Schaft 62 um die Verbindungsstelle mit dem Gehäuse 68 zu verschwenken. Durch die erhöhte Flexibilität der aufgenommenen Stablinsen 80 ist ein Brechen der Stablinsen stark verringert. Dies trägt zu einer erheblichen Steigerung der Lebensdauer des Endoskopes 60 bei.

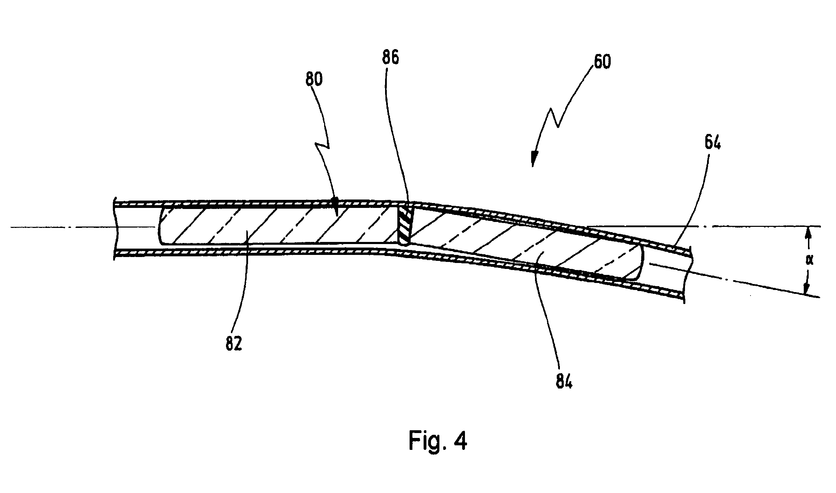

In Fig. 4 ist eine solche Situation dargestellt, d.h. der Schaft bzw. das Innenrohr 64 des Endoskops wurde im Bereich der Stablinse 80 verbogen. Eine konventionelle Stablinse aus Glas mit der Länge der Stablinse 80 wäre bei einer derart starken Krümmung gebrochen. Aufgrund der Flexibilität bzw. Elastizität des Zwischenstückes 86 sind solche Krümmungen ohne Bruch möglich.

Die Verbindungsstellen zwischen den glasförmigen Körpern und dem optischen Kitt wurden jeweils als planare Verbindungsstellen beschrieben.

Dies wird, wie zuvor erwähnt, hauptsächlich dann der Fall sein, wenn an sich in ihren optischen Eigenschaften bekannte Stablinsen durch eine Kittschicht zur Erhöhung der Flexibilität miteinander verbunden werden.

## Patentansprüche

1. Stablinse zum Einbau in Endoskope (60), mit einem stabförmigen Körper (12, 32) der zwei oder drei stabförmige, brüchige Elemente (14, 16; 34, 36, 38; 82, 84) aus Glas aufweist, wobei der Durchmesser der Elemente im Bereich von einigen Millimetern liegt und deren Länge mehrere Zentimeter beträgt, die jeweils durch ein flexibles, transparentes, massives Zwischenstück (18; 40, 42; 86) zu dem stabförmigen Körper (12, 32) zusammengesetzt sind, wobei die Länge eines Zwischenstückes klein gegenüber der Länge der Elemente ist,
wobei
das Zwischenstück (18; 40, 42; 86) aus einem, nach seiner Härtung, hochflexiblen optischen Kitt ausgebildet ist, und
der Kitt unmittelbar, ohne zusätzliche Klebemittel, mit den Elementen (14, 16; 34, 36, 38; 82, 84) aus Glas verbunden ist, so dass im Bereich der Zwischenstücke Biegestellen geschaffen sind, um die ein Abbiegen oder Abknicken der Elemente möglich ist, wobei die Verbindungsstellen (20, 22) der Elemente (14, 16; 34, 36, 38; 82, 84) planar sind.

2. Stablinse nach Anspruch 1, **dadurch gekennzeichnet, dass** der Körper (12) eine Knochenform aufweist.

3. Stablinse nach Anspruch 1 oder 2, **dadurch gekennzeichnet, dass** das flexible, transparente Kunststoffmaterial eine Shore Härte D kleiner 80, aufweist.

4. Endoskop (60), mit einem Optikrohr (64), in dem eine oder mehrere Stablinsen (80) nach einem der Ansprüche 1 bis 3 aufgenommen sind.

## Claims

1. Rod lens to be mounted in endoscopes (60), having a rod-shaped body (12, 32) comprising two or three rod-like, brittle elements (14, 16; 34, 36, 38; 82, 84) made of glass, a diameter of the elements is within a range of some millimeters and a length is several centimeters, which elements are assembled via a flexible, transparent, solid intermediate piece (18; 40, 42; 86) to the rod-shaped body (12, 32), wherein a length of the intermediate piece is small in comparison to the length of the elements, wherein the intermediate piece (18; 40, 42; 86) is made of an optical cement which, after curing, is highly flexible, and wherein the cement is joined to the elements (14, 16; 34, 36, 38; 82, 84) made of glass without an additional adhesive, thereby creating bending points in the area of the intermediate pieces about which the elements can be bent or kinked, and wherein joining points (20, 22) of the elements (14, 16; 34, 36, 38; 82, 84) are planar.

2. Rod lens of claim 1, **characterized in that** the body (12) has the shape of a bone.

3. Rod lens of claims 1 or 2, **characterized in that** the flexible, transparent synthetic material has a Shore Hardness D of less than 80.

4. Endoscope (60) with an optic tube (64) housing one or more of the rod lenses (80) of one of claims 1 through 3.

## Revendications

1. Lentille barreau destinée à être montée dans des endoscopes (60), possédant un corps (12, 32) en forme de barreau qui présente deux ou trois éléments cassants en forme de barreau (14, 16 ; 34, 36, 38 ; 82, 84) en verre, le diamètre des éléments étant de l'ordre de quelques millimètres et leur longueur étant de plusieurs centimètres, lesquels sont assemblés chaque fois par une pièce intermédiaire flexible, transparente et massive (18 ; 40, 42 ; 86) pour former le corps (12, 32) en forme de barreau, la longueur d'une pièce intermédiaire étant petite par rapport à la longueur des éléments,
la pièce intermédiaire (18 ; 40, 42 ; 86) étant formée d'un mastic optique à haute flexibilité après son durcissement, et
le mastic étant relié directement, sans adhésifs supplémentaires, aux éléments (14, 16 ; 34, 36, 38 ; 82, 84) en verre, de sorte que sont créés dans la région des pièces intermédiaires des points de flexion qui permettent une flexion ou un pliage des éléments, les zones de liaison (20, 22) des éléments (14, 16 ; 34, 36, 38 ; 82, 84) étant planes.

2. Lentille barreau selon la revendication 1, **caractérisée en ce que** le corps (12) présente une forme d'os.

3. Lentille barreau selon la revendication 1 ou 2, **caractérisée en ce que** la matière plastique flexible transparente présente une dureté Shore D inférieure à 80.

4. Endoscope (60) muni d'un tube optique (64) dans lequel sont logées une ou plusieurs lentilles barreau (80) selon une des revendications 1 à 3.
